# Europäisches Patentamt

# European Patent Office

# Office européen des brevets

(19)

(11) Veröffentlichungsnummer: **0 114 160**
**B1**

(12) ## EUROPÄISCHE PATENTSCHRIFT

(45) Veröffentlichungstag der Patentschrift:
23.09.87

(21) Anmeldenummer: 84890007.2

(22) Anmeldetag: 10.01.84

(51) Int. Cl.⁴: **C 13 K 1/06**, C 12 P 19/14

(54) **Verfahren zum enzymatischen Verzuckern von stärkehaltigen Rohstoffen.**

(30) Priorität: 13.01.83 AT 107/83

(43) Veröffentlichungstag der Anmeldung:
25.07.84 Patentblatt 84/30

(45) Bekanntmachung des Hinweises auf die Patenterteilung:
23.09.87 Patentblatt 87/39

(84) Benannte Vertragsstaaten:
DE FR SE

(56) Entgegenhaltungen:
DE-A-1 442 229
US-A-4 243 750

(73) Patentinhaber: **VOEST- ALPINE Aktiengesellschaft,
Friedrichstrasse 4, A-1011 Wien (AT)**

(72) Erfinder: **Cvitas, Vilim, Dipl.- Ing., Voltastrasse 29,
A-4040 Linz (AT)**
Erfinder: **Faltejsek, Karl, Dipl.- Ing.,
Lüfteneggerstrasse 6, A-4020 Linz (AT)**
Erfinder: **Hanke, Reinhart, Dipl.- Ing.,
Annaberggasse 2, A-8700 Leoben (AT)**
Erfinder: **Treso, Bertalan, Kunigundenweg 11a,
A-8707 Leoben (AT)**

(74) Vertreter: **Haffner, Thomas M., Dr.,
Patentanwaltskanzlei Dipl.- Ing. Adolf
Kretschmer Dr. Thomas M. Haffner
Schottengasse 3a, A-1014 Wien (AT)**

EP 0 114 160 B1

## Beschreibung

Die Erfindung bezieht sich auf ein Verfahren zum enzymatischen Verzuckern von stärkehaltigen Rohstoffen. Aus der EP-A 0 044 428 ist es bereits bekannt, zur Herstellung von Alkohol aus Stärke oder stärkehaltigen Rohstoffen eine Schlempe zu gewinnen, welche durch Zerkleinern, thermischen Aufschluß und Verzuckerung der stärkehaltigen Rohstoffe gebildet wird. Es sind eine Reihe von zuckerhaltigen Rohstoffen bekannt, aus welchen unmittelbar Zuckerlösungen auf extraktivem Weg hergestellt werden können, und es ist bereits bekannt, derartige Zuckerlösungen bis zur Glucose abzubauen und eine auf diese Weise erhaltene Maische zu vergären. Der Gärprozeß verlangt hierbei je nach den eingesetzten Rohstoffen bzw. der eingesetzten Maische unterschiedliche Bedingungen und erfordert einen relativ langen Reaktionszeitraum für den Abschluß der Gärung. Nachteilig bei den bekannten Gärverfahren ist hierbei die Tatsache, daß die Maischen bzw. gärfähigen Substrate im Verlauf der Gärung unterschiedliche Konzentrationen sowohl an vergärbarem Zucker als auch an Gärungsprodukten aufweisen, welche mit zunehmender Gärung die Reaktionsgeschwindigkeit bzw. die Gärungsgeschwindigkeit stark beeinflussen. Es ist weiters nachteilig, daß mit den bekannten Verfahren immer nur ein mehr oder minder reiner Alkohol und in der Regel mit geringer Konzentration hergestellt werden kann.

Das durch Verzuckerung erhältliche Zucker enthaltende Gärsubstrat weist je nach Ausbeute der Verzuckerung sowie in Abhängigkeit von der Qualität des stärkehaltigen Ausgangsmaterials unterschiedliche Zuckerkonzentrationen auf und auch diese unterschiedlichen Konzentrationen sind für eine rasche Verfahrensführung während des nachfolgenden Gärverfahrens besonders nachteilig. Die Erfindung zielt nun darauf ab, ein Gärsubstrat zu erhalten, welches unabhängig von der Ausbeute der Verzuckerung und von der Qualität der stärkehaltigen Rohstoffe eine gleichbleibende Zuckerkonzentration sicherstellt. Zur Lösung dieser Aufgabe besteht die Erfindung im wesentlichen darin, daß den stärkehaltigen Rohstoffen vor der Verzuckerung ein Teil der nach der Verzuckerung anfallenden von Feststoffen befreiten, auf höhere Konzentration gebrachten Zuckerlösung zugesetzt wird und daß die der Verzuckerung rückgeführte Teilmenge der Zuckerlösung in Abhängigkeit vom Stärkegehalt der Rohstoffe so gewählt wird, daß nach der Verzuckerung eine Zuckerlösung mit konstantem Zuckergehalt, insbesondere innerhalb des Bereiches von 15 bis 22 Gew.%, vorzugsweise etwa 20 Gew.%, erhalten wird.

Durch die Rückführung eines Teiles der erhaltenen Zuckerlösung läßt sich eine exakte Regelung der Konzentration erzielen. Wenn vor der Verzuckerung eine Verkleisterung bzw. thermische Konditionierung erfolgte, wird diese üblicherweise bei Temperaturen von rund 120°C durchgeführt. Der Zusatz der bereits von Feststoffen befreiten, auf höhere Konzentration gebrachten Zuckerlösung, welche naturgemäß bereits weitergehend abgekühlt ist, erlaubt gleichzeitig die Einstellung der für die Verzuckerung üblichen Temperaturen von rund 70°C unter gleichzeitiger Verbesserung der Energiebilanz. Das erfindungsgemäße Verfahren wird hiebei so durchgeführt, daß die der Verzuckerung rückgeführte Teilmenge der Zuckerlösung in Abhängigkeit vom Stärkegehalt der Rohstoffe so gewählt wird, daß nach der Verzuckerung Zuckerlösung mit konstantem Zuckergehalt, insbesondere innerhalb des Bereiches von 15 bis 22 Gew.%, vorzugsweise etwa 20 Gew.%, erhalten wird. Es wird somit gleichzeitig mit der Erzielung eines Gärsubstrates mit gleichbleibender Zuckerkonzentration auch die Energiebilanz des Verfahrens verbessert. Durch das Abtrennen der Feststoffe aus der rückzuführenden Zuckerlösung ergibt sich bereits eine Konzentrationserhöhung, welche naturgemäß durch Verdampfen bzw. Eindicken weiter gesteigert werden kann.

Es hat sich hiebei völlig überraschenderweise gezeigt, daß trotz Erhöhung des Zuckergehaltes am Beginn der Verzuckerung keine Enzymblockade auftritt und daß die enzymatische Verzuckerung durch den Zusatz von zuckerhaltiger Lösung in keiner Weise beeinträchtigt wird.

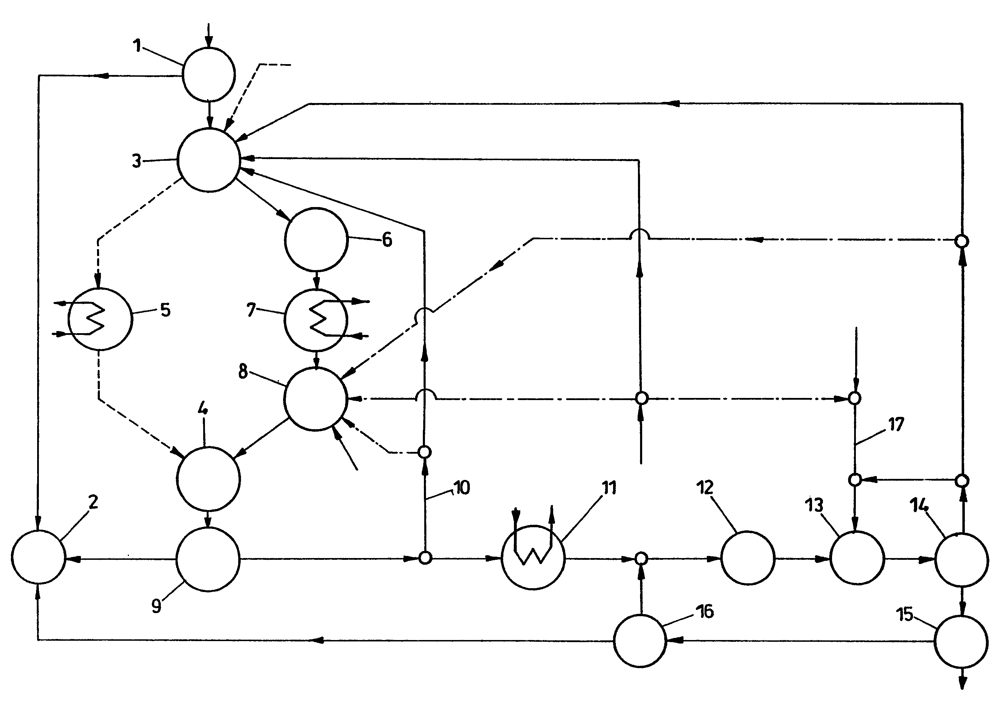

Die Erfindung wird nachfolgend an Hand eines in der Zeichnung dargestellten Ausführungsbeispieles näher erläutert.

Bei der im Flußdiagramm dargestellten Verfahrensweise wird in der ersten, mit 1 bezeichneten Stufe die mechanische Aufbereitung von Rohmais vorgenommen. Der Siebüberlauf, welcher in erster Linie die Schalen enthält, wird einer Futtermittelerzeugung 2 zugeführt. Das mechanisch aufbereitete Material wird bei 3 mit Wasser vermischt. Sofern anschließend unmittelbar die Verzuckerung 4 vorgenommen wird, wird gleichzeitig Enzymlösung zugeführt und die Lösung durch eine Heizung 5 auf eine Verzuckerungstemperatur von etwa 70°C gebracht. Wenn vor der Verzuckerung eine thermische Konditionierung erfolgen soll, kann vor der thermischen Konditionierung noch keine Enzymlösung zugesetzt werden, da diese bei den höheren Temperaturen des thermischen Aufschlusses zerstört würde. In diesem Falle wird die Mischung aus 3 bei 6 auf etwa 120°C erhitzt und thermisch aufgeschlossen und anschließend einer Kühlung 7 unterworfen, um die Bedingungen für die Verzuckerung einzustellen. In diesem Falle wird die Enzymlösung einer dieser Kühlung nachgeschalteten Mischstufe 8 zugeführt. Die verzuckerte Lösung wird einer Trennung 9 in feststofffreie Zuckerlösung und Schlammkuchen unterworfen, wobei der Schlammkuchen wiederum der Futtermittelerzeugung 2 zugeführt werden kann.

Aus dieser feststofffreien Zuckerlösung wird ein Teil über eine Leitung 10 der Mischstufe 3 rückgeführt. Im Falle der thermischen Konditionierung kann ein Teil dieser feststoffreien Zuckerlösung auch der zweiten thermischen Mischstufe 8 zugefügt werden. Der Hauptanteil der feststoffreien Zuckerlösung wird in der Folge bei 11 auf etwa 30°C gekühlt und mit Hefe versetzt einer Mischstufe 12 zugeführt. Mit der Mischung kann auch eine Flockung bei 13 und eine Flotation bei 14 vorgenommen werden, worauf der zuckerreiche Hefeschlamm der Gärung bei 15 unterworfen wird. Nach beendeter Gärung kann die Hefe einer Heferegenierierung 16 zugeführt werden, wobei die überschüssige Hefe in die Futtermittelproduktion 2 übergeführt und ein Teil der regenerierten Hefe wiederum der feststofffreien Zuckerlösung zugesetzt wird.

Für die Flockung wird über eine Leitung 17 Polyelektrolyt zugeführt und es kann in der Mischstufe 13, in welcher auch die Flockung vorgenommen wird, Restwasser aus der Flotation mitverwendet werden. Das Restwasser aus der Flotation wird zusammen mit Frischwasser in der Mischstufe 3 bzw. der Mischstufe 8 eingesetzt.

Auch das Restwasser aus der Flotation enthält vergärbaren Zucker und es wird daher der noch nicht der Gärung unterworfene vergärbare Zucker vollständig im Kreislauf geführt. Sowohl mit der feststofffreien Zuckerlösung, welche über die Leitung 10 abgezogen wird, als auch mit dem Restwasser aus der Flotation ergibt sich die Möglichkeit einer Regelung des Zuckergehaltes der nach dem Verzuckern bei 4 erhaltenen Zuckerlösung.

## Patentansprüche

Verfahren zum enzymatischen Verzuckern von stärkehaltigen Rohstoffen, dadurch gekennzeichnet, daß den stärkehaltigen Rohstoffen vor der Verzuckerung ein Teil der nach der Verzuckerung anfallenden von Feststoffen befreiten, auf höhere Konzentration gebrachten Zuckerlösung zugesetzt wird, und daß die der Verzuckerung rückgeführte Teilmenge der Zuckerlösung in Abhängigkeit vom Stärkegehalt der Rohstoffe so gewählt wird, daß nach der Verzuckerung eine Zuckerlösung mit konstantem Zuckergehalt, insbesondere innerhalb des Bereiches von 15 bis 22 Gew.%, vorzugsweise etwa 20 Gew.%, erhalten wird.

## Claim

A process for enzymatically saccharifying starch containing raw materials, characterised in that prior to saccharification a part of the sugar solution obtained after the saccharification step, having been separated from solid matter and having been brought to a higher concentration, is added to the starch containing raw materials and that the amount of the portion of sugar solution recycled to saccharification is selected in dependence on the starch content of the raw materials such that after saccharification a sugar solution having constant sugar content, particularly within the range of from 15 to 22 percent by weight, preferably about 20 percent by weight, is obtained.

## Revendication

Procédé de saccharification enzymatique de matières premières amylacées, caractérisé en ce que, aux matières premières amylacées, on ajoute, avant la saccharification, une partie de la solution sucrée, amenée à une concentration élevée, éventuellement débarrassée des substances solides après la saccharification, et en ce que la quantité de la solution sucrée remise en circulation dans la saccharification est choisie, en fonction de la teneur en amidon de la matière première de telle manière qu'après la saccharification on obtient une solution sucrée avec une teneur constante en sucre, en particulier comprise entre 15 et 22 % en poids, de préférence d'environ 20 % en poids.